# EUROPEAN PATENT SPECIFICATION

(11) **EP 2 890 985 B1**
(45) Date of publication and mention of the grant of the patent: **20.11.2019**
(21) Application number: 13786754.5
(22) Date of filing: 26.08.2013
(51) Int. Cl.: G01N 33/569, G16H 50/50, G01N 33/49

(54) **WHITE BLOOD CELL MONITORING DURING TREATMENT CYCLES**
ÜBERWACHUNG WEISSER BLUTZELLEN WÄHREND BEHANDLUNGSZYKLEN
SURVEILLANCE DES LEUCOCYTES PENDANT DES CYCLES DE TRAITEMENT

(30) Priority: 31.08.2012 US 201261695564 P
(43) Date of publication of application: 08.07.2015
(73) Proprietor: Koninklijke Philips N.V., 5656 AE Eindhoven (NL)
(72) Inventor: VAN BERKEL, Cees, NL-5656 AE Eindhoven (NL)
(74) Representative: Steffen, Thomas
(86) International application number: PCT/IB2013/056887
(87) International publication number: WO 2014/033610

(56) References cited:
- US-A1- 2007 027 636
- ELENA SOTO ET AL: "Semi-mechanistic population pharmacokinetic/pharmacodynamic model for neutropenia following therapy with the Plk-1 inhibitor BI 2536 and its application in clinical development", CANCER CHEMOTHERAPY AND PHARMACOLOGY, SPRINGER, BERLIN, DE, vol. 66, no. 4, 9 January 2010 (2010-01-09), pages 785-795, XP019843353, ISSN: 1432-0843
- SANDSTRÖM M ET AL: "Population analysis of the pharmacokinetics and the haematological toxicity of the fluorouracil-epirubicin-cyclophosphamide regimen in breast cancer patients", CANCER CHEMOTHERAPY AND PHARMACOLOGY, SPRINGER, BERLIN, DE, vol. 58, no. 2, 1 August 2006 (2006-08-01) , pages 143-156, XP019334382, ISSN: 1432-0843, DOI: 10.1007/S00280-005-0140-2

## Description

### TECHNICAL FIELD OF THE INVENTION

The invention relates to methods and apparatus for white blood cell monitoring during treatment cycles and in particular to methods and apparatus for predicting white blood cell counts, and especially neutrophil count.

### BACKGROUND TO THE INVENTION

Many chemotherapy treatments are often administered on an outpatient basis, with the treatment typically comprising a number of cycles of treatment. The duration of each cycle may typically be of the order of 2-3 weeks. During each cycle the chemotherapy drugs affect fast-dividing cells, such as tumour cells but also the pluripotent stem cells in the bone marrow that pre-stage blood cells. Chemotherapy treatment therefore also reduces the white blood cell count of a patient. If the white blood cell count drops too low, especially if the neutrophil counts drops and the patient experiences neutropenia, this can put the patient at increased risk of potentially life threatening infection and can result in delay of the next cycle of chemotherapy treatment with a possible reduction in efficacy of the treatment.

The counts of the different white blood cells drop to a lowest point, called nadir, during the cycle before hopefully recovering in time for the start of the next cycle. If the count at the nadir is too low, or if the patient develops a fever at or near the nadir, this is classed as an adverse event.

It has been proposed to monitor white blood cell count in support of chemotherapy treatments, in particular absolute neutrophil count (ANC). The total white blood cell count is made up of a number of sub populations, principally the neutrophils, lymphocytes and monocytes. In some instances ANC may be of main clinical interest. ANC may therefore be measured before the nadir is expected and an alert generated if below a certain threshold. However, to allow for preventative action to be taken if necessary a conservative threshold must be used, resulting in a large number of alerts requiring consideration by a healthcare professional.

ANC may also be measured prior to starting the next cycle and if the recovered count at the end of the cycle is too low to safely allow the next chemotherapy dose to be administered, the treatment may be delayed. However cancelling a planned treatment can cause scheduling issues for the patient and healthcare provider. A cancelled appointment at a late stage may result in non-optimal use of resources of the healthcare provider. Also the next opportunity for the patient to attend treatment, allowing sufficient time to ensure their cell count has recovered, may result in a delay before treatment that is longer than is actually necessary which may impact on overall treatment efficacy.

It is therefore desirable to provide improved methods of monitoring white blood cell count that are convenient for the patient, for use in the management of chemotherapy treatment.

### SUMMARY OF THE INVENTION

According to a first aspect of the invention, there is provided a method of predicting, for a subject, a cell count of at least one white blood cell component within a chemotherapy treatment cycle, the method comprising: taking, within said cycle, at least one measurement of a cell count of said white blood cell component of the subject; identifying at least one modeled trajectory of white blood cell count that matches said at least one measurement for said subject; and based on said at least one modeled trajectory identifying a likely cell count at a later date in the cycle and/or start of the next cycle, wherein the step of identifying at least one modeled trajectory comprises taking a reference set of trajectories suitable for the subject and identifying, from said reference set, a selection set of trajectories that matches said at least one measurement for said subject, wherein said reference set of trajectories are modeled for the subject by identifying a set of model parameters and parameter variances that correspond to the subject and modeling a trajectory for each instance of identified parameter values.

The step of identifying at least one modeled trajectory comprises taking a reference set of trajectories suitable for the subject and identifying, from said reference set, a selection set of trajectories that matches said at least one measurement for said subject.

The reference set of trajectories is modeled for the subject by identifying a set of model parameters and parameter variances that correspond to the subject and modeling a trajectory for each instance of identified parameter values. At least one subject parameter or variance may be based on population averages suitable for the subject.

The method may comprise predicting a cell count nadir. In which case the method may comprise determining the average nadir of the trajectories of the selection set as the predicted cell count nadir. An alert may be generated if the predicted cell count nadir is below a threshold level.

The method may additionally comprise at least one of: identifying the proportion of trajectories of the selection set having a nadir below a threshold level; and determining the spread of nadir values of the trajectories of the selection set.

The proportion of trajectories of the selection set having a nadir below a threshold level may be identified as a risk level. The spread of nadir values of the trajectories of the selection set may be identified as a confidence level.

The method may additionally or alternatively comprises identifying the cell count at or near the expected end of the current cycle and/or start of the next cycle.

The cell count may be the absolute neutrophil count.

The at least one modeled trajectory may be generated using a hematopoietic model.

At least one modeled trajectory of white blood cell count may model the subject having a treatment to stimulate white blood cell production.

The invention also relates to an apparatus for predicting a cell count of at least one white blood cell component. Thus in another aspect of the invention there is provided an apparatus for predicting, for a subject, a cell count of at least one white blood cell component within a chemotherapy treatment cycle, the apparatus comprising: a data input interface for receiving at least one measurement of a cell count of said white blood cell component of the subject; and a processor configured to identify at least one modeled trajectory of white blood cell count that matches said at least one measurement for said subject; and based on said at least one modeled trajectory identifying a likely cell count at a later date in the cycle and/or start of the next cycle.

The processor may be adapted to interrogate a database comprising a reference set of trajectories suitable for the subject so as to identify, from said reference set, a selection set of trajectories that that matches said at least one measurement for said subject.

The invention in a further aspect provides a computer program product comprising computer readable code that, when executed by a suitable computer or processor, is configured to cause the computer or processor to perform the method described above.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a flow chart of a method according to an embodiment of the invention;
Fig. 2 shows a plot of a selection set of cell count trajectories;
Fig. 3 shows a plot of ANC nadir predicted using the methods of the present invention against measured ANC;
Fig. 4 illustrates selection sets of predicted ANC trajectory both with and without a growth factor being administered; and
Fig. 5 illustrates a system according to an embodiment of the present invention.

### DETAILED DESCRIPTION OF THE PREFERRED EMBODIMENTS

Embodiments of the present invention use biophysical models, e.g. hematopoietic models, to provide prediction of cell count of at least one white blood cell component, for example absolute neutrophil count (ANC). In some embodiments a stochastic use of the model can be used to identify a number of possible future scenarios consistent with the available data and the identified scenarios can be analyzed to determine desired information such as the risk of neutropenia.

Various biophysical models are known that model the effect of chemotherapy on cell count for various white blood cell components. For example various biophysical models are described in "Population analysis of the pharmacokinetics and the haematological toxicity of the fluorouracil-epirubicin-cyclophosphamide regimen in breast cancer patients" by M. Sanstrom et al. Cancer Chemother Pharmacol vol 58 pp143x156. 2006, and also in"Semi-mechanistic population pharmacokinetic/ pharmacodynamic model for neutropenia following therapy with the Plk-1 inhibitor BI 2536 and its application in clinical development" by Elena Soto et al. Cancer Chemother Pharmacol DOI 10.1007/s00280-009-1223-2. 2010, the contents of which are hereby incorporated by reference thereto.

These models use subject parameters such as dose, renal function, pre-treatment ANC, body surface area and maturation mean transit time in order to model how cell count may change over time for a subject undergoing chemotherapy. Mathematically the model is defined by a set of linked first order differential equations which can be implemented in a suitable processor to determine a cell count trajectory, i.e. an indication of how cell count will change over time, given a particular set of parameters.

It should be noted that models, such as that proposed by Soto et al., are not necessarily specific to the neutrophil count and could be used to model lymphocyte count, monocytes count or total white blood cell count, provided the correct parameters are used. It will be appreciated that in reality the maturation process of these cell types is different and more sophisticated models have taken this into account (see for instance "A mathematical model of hematopoiesis I. Periodic chronic myelogenous leukemia" Caroline Colijn, Michael C Mackey [McGill] Journal of Theoretical Biology vol 237 pp117-132, 2005).

For many embodiments the model described by Soto et al. is sufficient and can be used to determine absolute neutrophil count (ANC) trajectory given a suitable set of input parameters. The description below will discuss modeling of ANC but it will be understood that in other embodiments the model may additionally or alternatively be used to model lymphocyte count, monocytes count and/or total while blood cell counts.

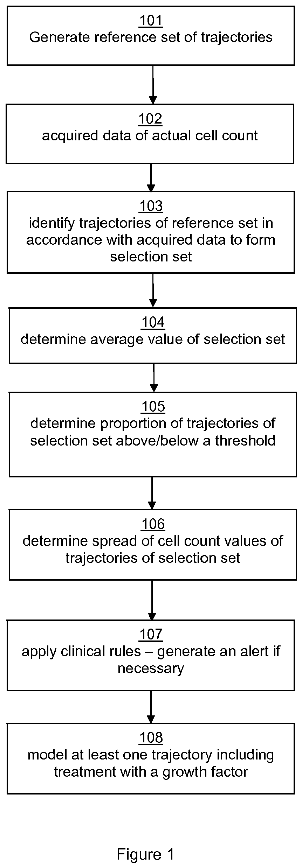

Figure 1 illustrates a flow chart illustrating one implementation of a method of predicting a nadir of cell counts according to the present invention.

At stage 101 a reference set of ANC trajectories may be generated for a subject, i.e. an individual undergoing a chemotherapy treatment. The reference set is preferably generated by taking parameters about a particular subject and their treatment regime which are known and estimating any model parameters that are not known based on suitable population averages for the subject in question. Thus parameters such as drug type and dose and age of the subject will generally be known. Parameters such as body surface area (if applicable) and/or pre-treatment neutrophil count may be known or may be specifically measured if required. Parameters such as clearance rate (or renal function) and transit time may not be known and thus may be estimated for the subject based on various population averages suitable for the particular subject. If parameters such as pre-treatment neutrophil count are not known these may also be estimated based on predetermined distribution characteristics. At least some variance for some of the parameters may also be determined using population variances that have previously been established. In other words a range of possible parameter values may be estimated. In some instances however a reference set that is suitable for a number of different subjects may be generated and the subject specific parameters used at a selection stage as will be described below. Typically the reference set is generated prior to the subject starting the treatment regime (and hence step 101 is shown as the initial step) but this is not always the case and the reference set may be generated at any suitable point in the process.

It should be noted that the distribution characteristics (e.g. average and standard deviation) of the model parameters, especially parameters such as clearance rate and transit time, may be taken from the existing literature and/or may have been previously established by measuring the actual ANC trajectory of a representative population of patients during treatment in order to determine the distribution characteristics. In use the parameter estimations and distribution characteristics can be updated as more and more cell count data is collected. In this way, if the measurement of cell count becomes regular practice the model parameters may be more accurately defined over time.

The reference set of trajectories may thus be modeled for the subject by identifying a set of model parameters and parameter variances that correspond to the subject and modeling a trajectory for each instance of identified parameter values. As mentioned at least one model parameter or variance may be based on population averages suitable for the subject.

In some embodiments the reference set may be generated to comprise of the order of about 1000 modeled trajectories although more or fewer trajectories could be included as desired.

In step 102 data regarding the actual cell count of the subject within a treatment cycle is acquired. Preferably this data is acquired by the subject self-testing at home using suitable measuring apparatus. Apparatus for measuring white blood cell count which is suitable for home use by the subject is known. Allowing a subject to self-test at home is convenient for both the subject and the health care provider. Typically the subject would therefore acquire at least one measure of their cell count, e.g. ANC, during the treatment cycle.

The cell count data may be used to determine an initial cell count trajectory. If the starting ANC at the start of, or just prior to, the current treatment cycle was known then one measurement of cell count may be sufficient to establish an initial trajectory. In some embodiments however the cell count may be determined at regular intervals at the start of the treatment cycle, for instance every one or two days.

As mentioned the measurements may be acquired by the subject self-testing in a convenient location such as the subject's home, although some subjects may require assistance with testing and in some instances the subject may visit a locations such as a local testing center to have measurements taken. The measurement schedule may involve more frequent measurements being acquired at certain parts the cycle. For instance more frequent measurements may be taken towards the beginning of a cycle to ensure the initial trajectory is well defined. Later in the cycle, when more data for that cycle is available, the requirements may be relaxed so as to reduce the measurement frequency for convenience of the subject. As mentioned the measurement frequency may be daily but in some instance more than one measurement may be acquired each day, at least for part of the treatment cycle. It will be appreciated that more measurements being acquired may allow more accurate identification of the initial cell count trajectory but may represent a greater testing burden for the subject.

To allow flexibility for the subject in acquiring the measurements, and also improve the accuracy of the results, the time at which a measurement is acquired may be recorded. Recording the time may allow measurements to be acquired at different times of the day to be used without skewing the results (the model time resolution or accuracy is such that time of day variations could have some impact if not correctly identified). The time of measurement could be recorded by a user, e.g. the subject, and/or the measurement apparatus may automatically record the time of measurement and add the time to the data record.

At step 103 the reference set of trajectories is examined to determine any trajectories in which the initial part of the trajectory matches the acquired data, within a desired tolerance. Those trajectories which match the initial data are identified to form a selection set of trajectories.

As the reference set may already have been chosen to match the parameters of the particular subject the step of identifying the selection set may simply involve matching the trajectories to the existing data. However in some instances some other parameter of the subject may be used to determine the trajectories that form the selection set. In addition any other bounding criteria may be used, for instance the ANC will not be negative. If however the reference set were a general population reference set which was not specific to the parameters of the particular subject the known parameters could be used at this stage to reduce the population reference set to a selection set appropriate for the subject.

The selection set of trajectories can then be used to predict cell count values at a later point in time in the same treatment cycle. It can be seen therefore that the present invention, in general, provides a method of predicting, for a subject, a cell count of at least one white blood cell component within a chemotherapy treatment cycle, which involves taking, within the cycle, at least one measurement of a cell count of said white blood cell component of the subject; identifying at least one modeled trajectory of white blood cell count that matches said at least one measurement for said subject; and based on said at least one modeled trajectory identifying a likely cell count at a later date in the cycle and/or start of the next cycle.

The method illustrated in figure 1 may be used to predict a cell count nadir time and ANC value as will be described below.

Having identified the selection set at step 103 the average cell count value of all the trajectories of the selection set may be determined at step 104. For predicting cell count nadir the average value will be an average of the nadir values of all the trajectories of the selection set. This average value may be taken as the prediction of the value of the nadir. The expected time of the nadir may also be determined by looking at the average of when the nadir occurs for the selection set of trajectories.

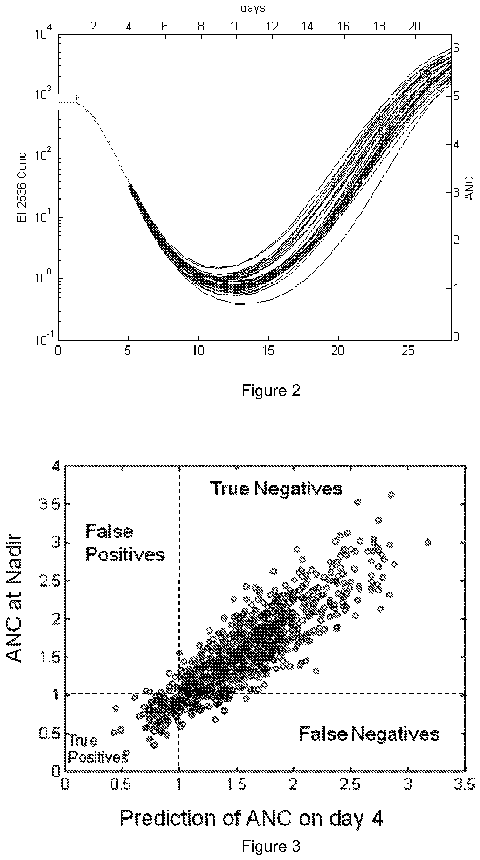

Figure 2 illustrates an example plot of a selection set of ANC trajectories. The reference set used to generate Figure 2 was a population data set for 10,000 subjects modeled using the hematopoietic model described by Sato et al. using the population data parameters and distribution characteristics described therein. This model was based on a 21-day treatment cycle of a known chemotherapy drug.

Actual ANC data for a given subject that had been acquired on days 2 and 4 of a cycle was taken and used to select from the population data set all trajectories that, within a set tolerance, had identical cell count values on days 2 and 4. This selection set was plotted as shown in Figure 2 (with cell count value being with respect to the right hand axis). In this example 30 trajectories were selected for the selection set.

It can be seen from Figure 2 that the selection set exhibits a range of cell count nadir values. For this example the average is 1.1 per nanolitre (nL) of blood.

The methods of the present invention thus enable a prediction of the nadir count value within a treatment cycle based on data acquired within that same cycle. The prediction can be made a number of days ahead and thus allows for early warning of any risk of neutropenia which may require preventative action but without undue false alarms. The ability to predict a value within the same treatment cycle as the data is acquired is a significant advantage of embodiments of the present invention.

Whilst the average value of nadir of the selection set may simply be used as the predicted count value, in some embodiments additional information may be determined by looking at the proportion of trajectories of the selection set with a nadir value less than a certain threshold and/or the spread of nadir values.

Thus, in addition to or instead of determining an average nadir value, the proportion of trajectories that that have a nadir count value below a threshold may be determined for one or more threshold levels at step 105 in Figure 1. By setting a threshold level and determining the proportion of trajectories of the selection set that have a nadir value below the threshold the relative risk of the subject's cell count dropping below the threshold can be determined.

Neutropenia is typically defined as an ANC of less than 1 per nanolitre (nL) of blood. Severe neutropenia is typically defined as less than 0.5/nL. Thus separate thresholds of an ANC of 1/nL and 0.5nL may be set. If, for example, 10% of the trajectories of the selection set have a nadir cell count of less than 1/nl this may be taken as a 10% risk that the count value may drop below 1/nL. It will be noted that the risk factor provides additional information as the predicted (average) nadir value may itself be above the threshold. Determining a risk factor in addition to a predicted average value improves the usefulness of the prediction.

In the example selection set shown in Figure 2 the proportion of trajectories with a nadir cell count of less than 1.0/nL (i.e. exhibiting neutropenia) is 19%.

Referring back to figure 1, optionally at step 106 the method may involve determining the spread, for example the standard deviation or other measure of distribution, of nadir values of the trajectories of the selection set. This may be taken as an indication of the confidence in the predicted nadir value. A narrow spread will indicate a higher confidence than a wide spread of possible nadir values.

At step 107 various clinical rules may be applied. For instance an alert may be automatically generated for a health care professional if predicted nadir value and/or risk factor exceed certain limit set by the health care professional. The rules may be based on predicted nadir value and/or risk factor and optionally may include confidence level. For instance an alert may be generated if the predicted average nadir value is below a first limit or the risk of reaching a second limit (which may be different to the first limit) exceeds a set value. In the event that an alert is generated the data may be forward to the health care professional along with an indication of the confidence value determined.

Note that the discussion above has focused on predicting cell count nadir. This is useful as it can provide information to a health care professional in time to decide whether any intervention is needed to prevent an adverse event. The method may also be used however to predict a cell count value at a set point in time or the time at which a set count value is likely to be reached. For example the method may involve determining a likely count value at the expected end of a treatment cycle and/or the expected time at which the cell count level will have recovered sufficiently for the next treatment cycle to commence.

In this example the method would operate in substantially the same way by comparing data acquired of actual cell count over the treatment cycle with the reference set of trajectories suitable for the subject to identify a selection set that match the acquired data. The selection set of trajectories could then be analyzed as required.

For instance the average value of cell count on a particular day could be determined at step 104, possibly along with the proportion of cell counts above or below a set threshold at step 105 and the spread of cell counts on that day at step 106. This may provide the predicted cell count for a particular day, say the day before the next treatment cycle is expected to begin, along with the risk factor that the cell count is not above a safe level and the confidence in the predicted value. This prediction may be made a few days before the next scheduled treatment thus allowing more time for a treatment to be re-scheduled if required. This may allow more efficient use of resources by the health care provider and also may reduce waiting time for the subject by allowing more time to schedule a new treatment date. An alert could be generated if the method predicted that the count value would not have recovered sufficiently by the scheduled start of the next cycle. The method may additionally or alternatively predict a date at which a safe cell count level will be reached by identifying the time at which the cell count level will exceed a threshold with a desired confidence level and/or acceptable risk level. This predicted date could be communicated to healthcare provider to allow appropriate scheduling of the next treatment.

The method of the present invention thus allows a healthcare professional to better manage a treatment regime for a subject, for example in setting treatment regimens, identifying any required interventions and making adjustments to proposed treatment intervals as required.

As described above in relation to Figure 1 the reference set may be generated at step 101, possibly using specific parameter values for the subject if known, and then at step 103 the trajectories of this reference set which match the acquired data may be identified. In some embodiments however the reference set may be generated taking any acquired measurement data into account and/or the method may involve re-generating the reference set during a treatment cycle taking any acquired measurement data into account.

For example an initial reference set may be generated to contain a desired number of reference trajectories based on suitable parameters and parameter variances. In some instances the initial trajectory determined by acquired cell count data may indicate that the actual trajectory is a relative outlier in the reference set, which may mean that the number of trajectories in the selection set is limited. Once the initially trajectory has been established the reference set could be re-generated, taking the initial trajectory into account to generate a reference set which has a greater number of trajectories that are near matches to the measured initial trajectory.

In some instance therefore an initial reference set may be used as a first level of selection, possibly with a first set of tolerances for matching the actual trajectory with a modeled trajectory, and once the initial trajectory is established a focused reference set may be generated with a greater number of modeled trajectories that may correspond to the acquired data. In some instances a second set of tolerances may be used be used to form the selection set from the focused reference set.

In some applications it may also be possible to re-generate a reference set for a subsequent treatment cycle based on data acquired from a previous cycle.

To evaluate the methods of the present invention a validation exercise was conducted wherein actual ANC data for 1000 subjects was analyzed. For each subject the data up to day 4 of the cycle was used to predict the nadir of ANC. The predicted ANC nadir was then compared to the actual ANC that was measured. The results are plotted in Figure 3. It can be seen that the predicted ANC nadir value corresponds well with the actual ANC nadir value measured.

Figure 3 also shows a threshold of ANC value of 1/nL and thus illustrates, were the predicted ANC nadir value to be used as an indicator of neutropenia, the number of true positives and negative (actual and predicted ANC nadir both less than 1/nL or both greater than 1/nL respectively) as well as the number of false negatives and false positives. Clearly the relative number of false positives and negatives could be adjusted by varying the threshold level at which an alert is generated. By using a threshold level which is higher than 1/nL the number of false negatives can be reduced (albeit at the cost of increasing the number of false positives).

A more useful figure of merit for a test however is the Area Under the Curve (AUC) of the operator receiver curve. An AUC of 1 represents a perfect test, while a value of 0.5 a random test. Table 1 below illustrates the value obtained in this case and also illustrates the effect of noise in the cell count measurements. Similar AUC values are obtained if the neutropenic risk is used as predictor.

**Table 1, Predictive Performance**

| | | | | |
|---|---|---|---|---|
| Noise | 1% | 5% | 15% | 30% |
| R2 | 87% | 79% | 55% | 49% |
| Sensitivity | 87% | 87% | 76% | 66% |
| Likelihood ratio | 6.7 | 6.7 | 3.1 | 1.9 |
| AUC | 0.95 | 0.93 | 0.83 | 0.74 |

It can be seen that the methods described above allow accurate prediction of nadir cell counts that can be used by a healthcare professional to inform treatment decisions. In particular if the predicted cell count nadir or risk factor indicates a possible risk of neutropenia a healthcare professional may decide to take preventative measures.

In clinical practice, growth factors, for example Granulocyte colony-stimulating factor (GCSF) drugs that stimulate the production of white blood cells, can be prescribed in cases when the clinician anticipates the occurrence of severe neutropenia. Standard usage of these drugs is either administration of multiple doses of a short lasting variant throughout the cycle or a single dose of a long lasting variant on the day following the administration of chemotherapy, on day 1 of the cycle.

The biophysical model used to generate the cell count trajectories can also model the effects of growth factors. In some embodiments therefore the method may involve, at step 108, identifying at least one modeled trajectory of white blood cell count that matches said at least one measurement for said subject and which models the subject having a treatment to stimulate white blood cell production. For instance the method may comprise the step of modeling the effect of introducing the Growth Factors on a predetermined day in the cycle.

Thus as described above ANC data may be acquired for a subject during a treatment cycle. The acquired data may be used to identify a selection set of trajectories that match the acquired data without any growth factor treatment. A set of trajectories corresponding to a growth factor treatment subsequently being applied may additionally be identified. The selection set corresponding to application of a growth factor may be determined as a matter or course or, in some embodiments, the effect of applying a growth factor may be identified only if the predicted nadir or risk factor without any growth factor meets certain criteria.

In this embodiment the effect of the chemotherapy treatment may be assessed within a cycle without having to initially decide whether a growth factor is required. For instance as discussed above an assessment could be made around day 4 of a 21 day cycle. At this time a reasonable prediction can be made on the depth and timing of the nadir but there will still be sufficient time for growth factor to be applied if required. It is then possible to present the clinician with values for the nadir in two scenarios. One scenario in which growth factors are not administered and one scenario in which growth factors are administered, for example starting from day 4.

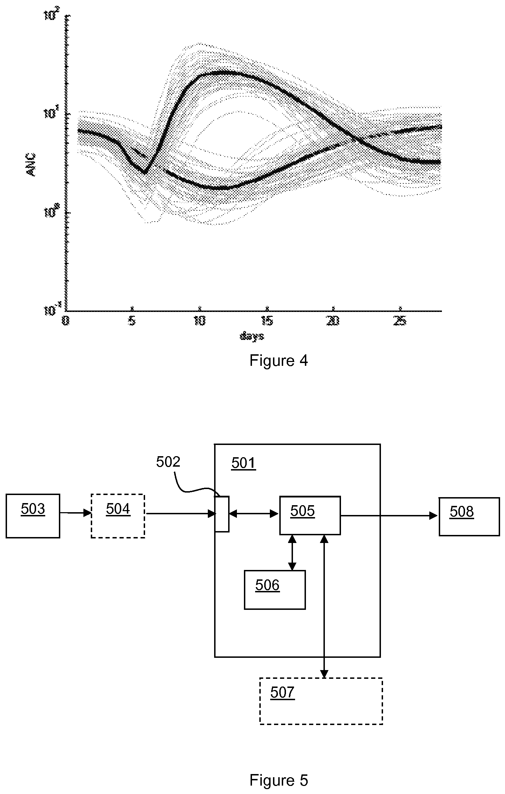

Figure 4 illustrates in general two sets, one corresponding to ANC count without any growth factor applied and the other corresponding to a treatment to activate white blood cell production at day 4.

Use of biophysical models to model to model the effect of treatments to stimulate white blood cell count during a treatment cycle represents another innovative aspect of the embodiments of the present invention.

Figure 5 illustrates a system for according to an embodiment of the invention. Figure 5 shows an apparatus 501 for predicting, for a subject, a cell count of at least one white blood cell component within a chemotherapy treatment cycle. The apparatus 501 has a data input interface 502 for receiving at least one measurement of a cell count of said white blood cell component of the subject. The data may be acquired using a white blood cell count measuring apparatus 503, such as that described in patent publication WO03/100402 or any suitable apparatus for determining a level of at least one white blood cell component. Preferably measuring apparatus 503 is used at the home of the subject and may be adapted so that the subject can self-test. In some instances the apparatus 501 for predicting cell count and measuring apparatus 503 may form part of the same apparatus, or may be both located in the subjects home, but in other embodiments the apparatus 501 may be remotely located from the measuring apparatus 503 and thus measuring apparatus 503 may be configured for remote transfer of data, possible via an intermediate device 504 such as a hub device or a computing device or communications device (e.g. mobile telephone) of the subject. Data transfer may be by any suitable means such as via the internet for example.

The apparatus 501 has a processor 505. The processor may be arranged to interrogate a database 506 comprising a reference set of trajectories suitable for the subject so as to identify, from said reference set, a selection set of trajectories that that matches said at least one measurement for said subject. The database comprising the reference set may be stored within, or generated as required by, apparatus 501 which may for instance form part of a healthcare system. In other embodiments however there may be an external database 507 and model which is accessed as required by the apparatus 501. Based on the reference set of modeled trajectories and acquired data the processor 505 identifies a likely cell count at a later date in the cycle and/or start of the next cycle, for example a predict nadir cell count and/or risk that the nadir cell count is below a set threshold.

The apparatus 501 may then apply one or more clinical rules set by a healthcare professional to determine whether the predicted cell count or risk factor represents a possible problem as discussed above. Based on these rules the processor may transmit an alert to an apparatus 508 of a healthcare professional to indicate a possible problem.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of predicting, for a subject, a cell count of at least one white blood cell component within a chemotherapy treatment cycle, the method comprising:
taking, within said cycle, at least one measurement of a cell count of said white blood cell component of the subject;
identifying at least one modeled trajectory of white blood cell count that matches said at least one measurement for said subject; and
based on said at least one modeled trajectory identifying a likely cell count at a later date in the cycle and/or start of the next cycle,
wherein the step of identifying at least one modeled trajectory comprises taking a reference set of trajectories suitable for the subject and identifying, from said reference set, a selection set of trajectories that matches said at least one measurement for said subject, wherein said reference set of trajectories are modeled for the subject by identifying a set of model parameters and parameter variances that correspond to the subject and modeling a trajectory for each instance of identified parameter values.

2. A method as claimed in claim 1 wherein at least one subject parameter or variance is based on population averages suitable for the subject.

3. A method as claimed in any of claims 1 and 2 wherein the method comprises predicting a cell count nadir and the method comprises determining the average nadir of the trajectories of the selection set as the predicted cell count nadir.

4. A method as claimed in claim 3 comprising generating an alert if the predicted cell count nadir is below a threshold level.

5. A method as claimed in claim 3 or claim 4 where the method further comprises at least one of:
identifying the proportion of trajectories of the selection set having a nadir below a threshold level; and
determining the spread of nadir values of the trajectories of the selection set.

6. A method as claimed in claim 5 wherein the proportion of trajectories of the selection set having a nadir below a threshold level is identified as a risk level and/or the spread of nadir values of the trajectories of the selection set is identified as a confidence level.

7. A method as claimed in any preceding claim wherein the method comprises identifying the cell count at or near the expected end of the current cycle and/or start of the next cycle.

8. A method as claimed in any preceding claim wherein the cell count of at least one white blood cell component comprises the absolute neutrophil count.

9. A method as claimed in any preceding claim wherein the at least one modeled trajectory is generated using a hematopoietic model.

10. A method as claimed in any preceding claim comprising identifying at least one modeled trajectory of white blood cell count that matches said at least one measurement for said subject and which models the subject having a treatment to stimulate white blood cell production.

11. An apparatus for predicting, for a subject, a cell count of at least one white blood cell component within a chemotherapy treatment cycle, the apparatus comprising:
a data input interface for receiving at least one measurement of a cell count of said white blood cell component of the subject; and
a processor configured to identify at least one modeled trajectory of white blood cell count that matches said at least one measurement for said subject; and
based on said at least one modeled trajectory identifying a likely cell count at a later date in the cycle and/or start of the next cycle, wherein the step of identifying at least one modeled trajectory comprises taking a reference set of trajectories suitable for the subject and identifying, from said reference set, a selection set of trajectories that matches said at least one measurement for said subject, wherein said reference set of trajectories are modeled for the subject by identifying a set of model parameters and parameter variances that correspond to the subject and modeling a trajectory for each instance of identified parameter values.

12. An apparatus as claimed in claim 11 wherein said processor is adapted to interrogate a database comprising a reference set of trajectories suitable for the subject so as to identify, from said reference set, a selection set of trajectories that that matches said at least one measurement for said subject.

13. An apparatus as claimed in claim 12 wherein said processor is further adapted to predict a cell count nadir and to determine the average nadir of the trajectories of the selection set of trajectories as the predicted cell count nadir.

14. An apparatus as claimed in claim 13 wherein the apparatus is further arranged to generate an alert if the predicted cell count nadir is below a threshold level.

15. A computer program product comprising computer readable code that, when executed by a suitable computer or processor, is configured to cause the computer or processor to perform the method described in any of claims 1 - 10.

## Patentansprüche

1. Verfahren zum Vorhersagen einer Zellzahl von mindestens einer Leukozytenkomponente innerhalb eines Chemotherapie-Behandlungszyklus für einen Patienten, das Verfahren umfassend:
Durchführen mindestens einer Messung der Zellzahl der Leukozytenkomponente des Patienten innerhalb des Zyklus;
Identifizieren mindestens einer modellierten Trajektorie der Leukozytenzahl, die mit der mindestens einen Messung für den Patienten übereinstimmt; und
basierend auf der mindestens einen modellierten Trajektorie, Identifizieren einer wahrscheinlichen Zellzahl zu einem späteren Zeitpunkt in dem Zyklus und/oder zu Beginn des nächsten Zyklus,
wobei der Schritt des Identifizierens mindestens einer modellierten Trajektorie das Heranziehen eines Referenzsatzes von Trajektorien, die für den Patienten geeignet sind, und das Identifizieren eines Auswahlsatzes von Trajektorien aus dem Referenzsatz umfasst, der mit der mindestens einen Messung für den Patienten übereinstimmt, wobei der Referenzsatz von Trajektorien für den Patienten modelliert wird, indem ein Satz von Modellparametern und Parametervarianzen, die dem Patienten entsprechen, identifiziert und eine Trajektorie für jedes Vorkommen von identifizierten Parameterwerten modelliert wird.

2. Verfahren nach Anspruch 1, wobei mindestens ein Patientenparameter oder eine Varianz auf Bevölkerungsdurchschnitten basiert, die für den Patienten geeignet sind.

3. Verfahren nach einem der Ansprüche 1 und 2, wobei das Verfahren das Vorhersagen eines Zellzahl-Nadirs umfasst und das Verfahren das Bestimmen des durchschnittlichen Nadirs der Trajektorien der Auswahlmenge als den vorhergesagten Zellzahl-Nadir umfasst.

4. Verfahren nach Anspruch 3, umfassend das Erzeugen eines Alarms, wenn der vorhergesagte Zellzahl-Nadir unter einem Schwellenniveau liegt.

5. Verfahren nach Anspruch 3 oder Anspruch 4, wobei das Verfahren weiter mindestens eines umfasst von:
Identifizieren des Anteils der Trajektorien des Auswahlsatzes, die einen Nadir unterhalb eines Schwellenniveaus aufweisen; und
Bestimmen der Verteilung der Nadirwerte der Trajektorien des Auswahlsatzes.

6. Verfahren nach Anspruch 5, wobei der Anteil der Trajektorien der Auswahlmenge, die einen Nadir unterhalb eines Schwellenniveaus aufweisen, als ein Risikoniveau und/oder die Verteilung der Nadirwerte der Trajektorien der Auswahlmenge als Konfidenzniveau identifiziert wird.

7. Verfahren nach einem der vorstehenden Ansprüche, wobei das Verfahren das Identifizieren der Zellzahl zum oder nahe dem erwarteten Ende des aktuellen Zyklus und/oder dem Beginn des nächsten Zyklus umfasst.

8. Verfahren nach einem der vorstehenden Ansprüche, wobei die Zellzahl von mindestens einer Leukozytenkomponente die absolute Neutrophilenzahl umfasst.

9. Verfahren nach einem der vorstehenden Ansprüche, wobei die mindestens eine modellierte Trajektorie unter Verwendung eines hämatopoetischen Modells erzeugt wird.

10. Verfahren nach einem der vorstehenden Ansprüche, umfassend das Identifizieren mindestens einer modellierten Trajektorie der Leukozytenzahl, die mit der mindestens einen Messung für den Patienten übereinstimmt und die den Patienten modelliert, der eine Behandlung zur Stimulierung der Leukozytenproduktion aufweist.

11. Einrichtung zum Vorhersagen einer Zellzahl von mindestens einer Leukozytenkomponente innerhalb eines Chemotherapie-Behandlungszyklus für einen Patienten, die Einrichtung umfassend:
eine Dateneingabeschnittstelle zum Empfangen von mindestens einer Messung der Zellzahl der Leukozytenkomponente des Patienten; und
einen Prozessor, der konfiguriert ist, um mindestens eine modellierte Trajektorie der Leukozytenzahl zu identifizieren, die mit der mindestens einen Messung für den Patienten übereinstimmt; und
basierend auf der mindestens einen modellierten Trajektorie, Identifizieren einer wahrscheinlichen Zellzahl zu einem späteren Zeitpunkt in dem Zyklus und/oder zu Beginn des nächsten Zyklus, wobei der Schritt des Identifizierens mindestens einer modellierten Trajektorie das Heranziehen eines Referenzsatzes von Trajektorien, die für den Patienten geeignet sind, und das Identifizieren eines Auswahlsatzes von Trajektorien aus dem Referenzsatz umfasst, der mit der mindestens einen Messung für den Patienten übereinstimmt, wobei der Referenzsatz von Trajektorien für den Patienten modelliert wird, indem ein Satz von Modellparametern und Parametervarianzen, die dem Patienten entsprechen, identifiziert und eine Trajektorie für jedes Vorkommen von identifizierten Parameterwerten modelliert wird.

12. Einrichtung nach Anspruch 11, wobei der Prozessor angepasst ist, um eine Datenbank abzufragen, die einen Referenzsatz von Trajektorien umfasst, die für den Patienten geeignet sind, um aus dem Referenzsatz einen Auswahlsatz von Trajektorien zu identifizieren, der mit der mindestens einen Messung für den Patienten übereinstimmt.

13. Einrichtung nach Anspruch 12, wobei der Prozessor weiter angepasst ist, um einen Zellzahl-Nadir vorherzusagen und den durchschnittlichen Nadir der Trajektorien des Auswahlsatzes von Trajektorien als den vorhergesagten Zellzahl-Nadir zu bestimmen.

14. Einrichtung nach Anspruch 13, wobei die Einrichtung weiter angeordnet ist, um einen Alarm zu erzeugen, wenn der vorhergesagte Zellzahl-Nadir unter einem Schwellenniveau liegt.

15. Computerprogrammprodukt, umfassend einen computerlesbaren Code, der, wenn er von einem geeigneten Computer oder Prozessor ausgeführt wird, konfiguriert ist, um den Computer oder Prozessor zu veranlassen, das in einem der Ansprüche 1-10 beschriebene Verfahren durchzuführen.

## Revendications

1. Procédé de prédiction pour un sujet d'une numération globulaire d'au moins un composant leucocytaire dans un cycle de traitement de chimiothérapie, le procédé comprenant :
le prélèvement dans ledit cycle d'au moins une mesure d'une numération globulaire dudit composant leucocytaire du sujet ;
l'identification d'au moins une trajectoire modélisée de numération leucocytaire qui coïncide avec ladite au moins une mesure pour ledit sujet ; et,
sur la base de ladite au moins une trajectoire modélisée, l'identification d'une numération globulaire probable à une date ultérieure du cycle et/ou au début du cycle suivant,
dans lequel l'étape d'identification d'au moins une trajectoire modélisée comprend le prélèvement d'un ensemble de référence de trajectoires convenant au sujet et l'identification, à partir dudit ensemble de référence, d'un ensemble de sélection de trajectoires qui coïncide avec ladite au moins une mesure pour ledit sujet, dans lequel ledit ensemble de référence de trajectoires est modélisé pour le sujet par identification d'un ensemble de paramètres modèles et de variances paramétriques qui correspondent au sujet et modélisation d'une trajectoire pour chaque cas de valeurs paramétriques identifiées.

2. Procédé selon la revendication 1, dans lequel au moins un paramètre ou une variance du sujet est basé(e) sur des moyennes de population convenant au sujet.

3. Procédé selon l'une quelconque des revendications 1 et 2, dans lequel le procédé comprend la prédiction d'un nadir de numération globulaire et le procédé comprend la détermination du nadir moyen des trajectoires de l'ensemble de la sélection en tant que nadir de numération globulaire prédit.

4. Procédé selon la revendication 3, comprenant la génération d'une alerte si le nadir de numération globulaire prédit se situe en dessous d'un niveau de seuil.

5. Procédé selon la revendication 3 ou la revendication 4, dans lequel le procédé comprend en outre au moins l'un des cas suivants :
l'identification de la proportion de trajectoires de l'ensemble de sélection ayant un nadir en dessous d'un niveau de seuil ; et
la détermination de l'extension de valeurs de nadir des trajectoires de l'ensemble de sélection.

6. Procédé selon la revendication 5, dans lequel la proportion de trajectoires de l'ensemble de sélection ayant un nadir en dessous d'un niveau de seuil est identifiée comme niveau de risque et/ou l'extension de valeurs de nadir des trajectoires de l'ensemble de la sélection est identifiée comme niveau de confiance.

7. Procédé selon l'une quelconque des revendications précédentes, dans lequel le procédé comprend l'identification de la numération globulaire au niveau ou à proximité de l'extrémité attendue du cycle courant et/ou au début du cycle suivant.

8. Procédé selon l'une quelconque des revendications précédentes, dans lequel la numération globulaire d'au moins un composant leucocytaire comprend le taux absolu des polynucléaires neutrophiles.

9. Procédé selon l'une quelconque des revendications précédentes, dans lequel la au moins une trajectoire modélisée est générée en utilisant un modèle hématopoïétique.

10. Procédé selon l'une quelconque des revendications précédentes, comprenant l'identification d'au moins une trajectoire modélisée d'une numération globulaire leucocytaire qui coïncide avec ladite au moins une mesure pour ledit sujet et qui modélise le sujet ayant un traitement pour stimuler la production de leucocytes.

11. Appareil de prédiction pour un sujet d'une numération globulaire d'au moins un composant leucocytaire dans un cycle de traitement de chimiothérapie, l'appareil comprenant :
une interface d'entrée de données pour recevoir au moins une mesure d'une numération globulaire dudit composant leucocytaire du sujet ; et
un processeur configuré pour identifier au moins une trajectoire modélisée de numération leucocytaire qui coïncide avec ladite au moins une mesure pour ledit sujet ; et,
sur la base de ladite au moins une trajectoire modélisée identifiant une numération globulaire probable à une date ultérieure du cycle et/ou au début du cycle suivant, dans lequel l'étape d'identification d'au moins une trajectoire modélisée comprend le prélèvement d'un ensemble de référence de trajectoires convenant au sujet et l'identification à partir dudit ensemble de référence d'un ensemble de sélection de trajectoires qui coïncide avec ladite au moins une mesure pour ledit sujet, dans lequel ledit ensemble de référence de trajectoires est modélisé pour le sujet par identification d'un ensemble de paramètres modèles et de variances paramétriques qui correspondent au sujet et modélisation d'une trajectoire pour chaque cas de valeurs paramétriques identifiées.

12. Appareil selon la revendication 11, dans lequel ledit processeur est à même d'interroger une base de données comprenant un ensemble de référence de trajectoires convenant au sujet de manière à identifier à partir dudit ensemble de référence un ensemble de sélection de trajectoires qui coïncide avec ladite au moins une mesure pour ledit sujet.

13. Appareil selon la revendication 12, dans lequel ledit processeur est en outre à même de prédire un nadir de numération globulaire et de déterminer le nadir moyen des trajectoires de l'ensemble de sélection de trajectoires comme nadir de numération globulaire prédit.

14. Appareil selon la revendication 13, dans lequel l'appareil est en outre à même de générer une alerte si le nadir de numération globulaire prédit se situe en dessous d'un niveau de seuil.

15. Produit de programme d'ordinateur comprenant un code lisible par ordinateur qui, lorsqu'il est exécuté par un ordinateur ou un processeur approprié, est configuré pour amener l'ordinateur ou le processeur à effectuer le procédé décrit dans l'une quelconque des revendications 1 à 10.
